**Europäisches Patentamt**

**European Patent Office**

**Office européen des brevets**

(19)

(11) Veröffentlichungsnummer : **0 185 864 B2**

(12) # NEUE EUROPÄISCHE PATENTSCHRIFT

(45) Veröffentlichungstag der neuen Patentschrift :
**29.01.92 Patentblatt 92/05**

(51) Int. Cl.⁵ : **A61M 25/00**

(21) Anmeldenummer : **85112848.8**

(22) Anmeldetag : **10.10.85**

---

(54) **Punktions- und Einführungsbesteck.**

(30) Priorität : **14.12.84 DE 8436618 U**

(43) Veröffentlichungstag der Anmeldung :
**02.07.86 Patentblatt 86/27**

(45) Bekanntmachung des Hinweises auf die
Patenterteilung :
**11.01.89 Patentblatt 89/02**

(45) Bekanntmachung des Hinweises auf die
Entscheidung über den Einspruch :
**29.01.92 Patentblatt 92/05**

(84) Benannte Vertragsstaaten :
**AT BE CH DE FR GB IT LI LU NL SE**

(56) Entgegenhaltungen :
**EP-A- 0 051 718
DE-A- 2 305 640
FR-A- 2 316 972
FR-A- 2 474 317**

(56) Entgegenhaltungen :
**JP-U- 5 644 710
JP-U- 5 753 523
JP-U- 5 910 967
US-A- 4 235 232
Der Grosse Brockhaus, 1957, 11. Band (Sol-
Unj), Zitat "Stopfbüchse"
Hütte, des Ingenieurs Taschenbuch, 1949, Seiten 86 bis 89.**

(73) Patentinhaber : **B. Braun-SSC AG
Gerliswilstrasse 74
CH-6020 Emmenbrücke (CH)**

(72) Erfinder : **Haacke, Claus, Dr. Dipl.-Chem.
Altstadt 6
W-3508 Melsungen (DE)**
Erfinder : **Gäck Michael, Dr.med.
Ludwig-Konrad-Strasse 14
W-3590 Bad Wildungen (DE)**

(74) Vertreter : **von Kreisler, Alek, Dipl.-Chem. et al
Deichmannhaus am Hauptbahnhof
W-5000 Köln 1 (DE)**

EP 0 185 864 B2

**Beschreibung**

Die Erfindung bezieht sich auf ein Punktions- und Einführungsbesteck für einen Katheter oder dergleichen, mit Merkmalen des im Oberbegriff des Patentanspruchs 1 genannten Einführungsbesteckes.

Die punktion von Körperhohlräumen, z. B. Nierenbecken, Gallengang, Blase, Venen und Arterien geschieht sehr häufig zum nachträglichen Einlegen eines Katheters oder einer Sonde. Um bei der Punktion zu verhindern, daß Gewebe in das Lumen der Stahlkanüle eintritt und dieses während der Punktion verstopft, ist es üblich, in die Stahlkanüle einen Mandrin einzusetzen, der das Lumen der Stahlkanüle ausfüllt und im allgemeinen gleiche Länge wie die Stahlkanüle hat. Da der Durchmesser des einzuführenden Katheters oder der Sonde häufig größer ist als der Durchmesser der zur Punktion benutzten Stahlkanüle, ist eine Dilatation des Stichkanals erforderlich, was entweder mit der konisch angeformten Katheterspitze oder mit mindestens einem Dilatator erfolgt. Um nun das Punktionsloch in der Wand des Hohlraumes und auch den Stichkanal nach Zurückziehen der Stahlkanüle wiederzufinden, wird nach Herausziehen des Mandrins durch die Stahlkanüle ein drahtförmiger Führungsteil eingeschoben, der ein Seldingerdraht, ein Lunderquist-Draht oder dergleichen sein kann. Dieser Führungsteil bleibt mit seiner Spitze in dem Hohlraum liegen, während sein patientenfernes Ende aus der Haut herausragt. Die Punktionskanüle wird über den Draht zurückgezogen. Danach muß in einem fünften Schritt der Dilatator bzw. der Katheter über den Führungsteil in den Hohlraum vorgeschoben werden. Erst dann wird der Führungsteil entfernt. Dieses bisher geübte Vorgehen ist umständlich. Außerdem besteht die Gefahr, daß man nach Herausziehen des einen Teiles aus der Punktionskanüle und vor Einführung des nächsten Teiles in die Punktionskanüle Luft oder Kontaminationen in das Gefäß einschleppt oder daß man z. B. durch Bewegungen des Patienten und des Organs, wie z. B. Leber oder Niere, mit der Punktionskanüle aus dem punktierten Organ herausrutscht. Diesen Mangel kann auch das eingangs erwähnte Einführungsbesteck (EP-A-51 718) nicht beheben, das ausschließlich zum Einführen von Kathetern und dergleichen bestimmt ist und nicht gleichzeitig ein Punktionsbesteck bildet.

Der gummielastische Pfropfen des bekannten Einführungsbestecks besitzt einen dünnen membranartigen Mittelbereich, in dem ohne Materialentnahme ein Durchlaß ausgebildet ist. Auf den Pfropfen folgt koaxial ein hutförmiges Dichtungselement, dessen abgerundete Spitze zur Bildung von zwei Dichtlippen geschlitzt ist. Eine mit dem Gehäuse fest verbundene Kappe hält über eine gelochte Halteplatte die axial aufeinanderfolgenden Teile unveränderlich zusammen. Der Durchlaß im Pfropfen und die Dichtlippen des hutförmigen Dichtungselementes üben auf einen die Vorrichtung passierenden langgestreckten Teil einen leichten Druck aus, der zur Abdichtung genügt, jedoch die Verschiebung des Teiles nicht behindert. Punktieren kann man mit dem Einführungsbesteck nicht, weil das Einführungsrohr keine Punktionskanüle ist und weil der Führungsteil nicht axial unverschieblicher Mandrin in dem Einführungsrohr blockierbar ist.

Inzwischen ist ein Punktions- und Einführungsbesteck entwickelt worden (Arrow arterial catheterization system EP-A-93 164), bei dem ein drahtförmiger Führungsteil für den Katheter oder dergleichen in einem Ansatzstück einer Stahlkanüle verschiebbar vorgesehen ist und bei dem nach erfolgreicher Punktion bei zurückgezogenem Führungsteil Körperfluid zu einem durchsichtigen Kanülenansatz zurückströmen und das Punktionsergebnis dort anzeigen kann. Bei diesem Punktions- und Einführungsbesteck entfällt zwar der Schritt des Austausches eines Mandrins gegen den Führungsteil, jedoch treten Probleme während der Punktion dadurch auf, daß der Führungsteil von Hand in Stellung gehalten werden muß, wenn er als Mandrin für die Stahlkanüle dienen soll, wobei nicht gewährleistet ist, daß zuverlässig seine Spitze mit dem hinteren Rand des Stahlkanülenschliffs abschließt, um ein Eindrigen von Gewebe in das Stahlkanülenlumen zu verhindern. Außerdem ist es nicht ausgeschlossen, daß während des Vorschubes des Führungsteiles Luft in das Gefäß eingeschleppt wird oder daß die Punktions-kanüle aus dem punktierten Organ herausrutscht.

Ferner ist aus JP-Y-56-44710 eine "Schlauchnadel" bekannt, die aus drei ineinandersteckenden, relativ zueinander verschiebbaren flexiblen Schläuchen und Injektionsnadeln besteht und die dazu dient, nacheinander unterschiedliche Präparate im Bereich der Speiseröhre zu injizieren. Jeder Schlauch ist mit einem Schlauchansatzstück versehen, und die beiden inneren Schläuche tragen an ihrem proximalen Ende je eine Injektionskanüle. Um die beiden Injektionskanülen an dem inneren und dem mittleren Schlauch bei der Einführung der "Schlauchnadel" in die Speiseröhre beliebig gestaffelt zurückgezogen zu halten, werden die beiden Schläuche mittels eines durch axiale Pressung radial zusammenquetschbaren Pfropfens am Schlauchansatzstück des mittleren Schlauches miteinander verriegelt. Am Zielort werden die gekuppelten Schläuche gemeinsam vorgeschoben bis die Injektionskanüle am mittleren Schlauch über die Spitze des äußersten Schlauches vortritt und eine Injektion ermöglich. Nach Lösen der Verriegelung wird der innere Schlauch vorgeschoben, und durch seine Injektionskanüle kann eine weitere Injektion erfolgen. Zur Verwendung als Punktions- und Einführungsbesteck für einen Katheter ist die "Schlauchnadel" nicht geeignet, weil sie weder eine ausstanzungsfreie Punktion mit Anzeige des Punktionserfolges ermöglicht, noch das Einschleppen von Luft durch einen Schlauch in den Punktionshohlraum verhindert.

Der Erfindung liegt die Augabe zugrunde, ein Punktions- und Einführungsbesteckt mit einem drahtförmigen Führungsteil zu schaffen, bei dem der Abschluß der Spitze des Führungsteiles mit dem hinteren Rand eines Stahlkanülenschliffs bei der Punktion gewährleistet ist und in jeder Anwendungsphase das Einschleppen von Luft in den punktierten Körperholraum bzw. das unkontrollierte Ausfließen von Körperfluid durch den Kanülenansatz nach außen ausgeschlossen ist.

Diese Aufgabe wird erfindungsgemäß durch die Merkmale des Anspruchs 1 gelöst.

Der freie innere Rand des Hohlstutzens wird bei Aufschrauben des Schraubringes auf das Gehäuse beliebig fest gegen den Pfropfen gepreßt und dieser verformt sich, wobei der Durchlaß radial verengt wird. Der gummielastische Pfropfen in dem Gehäuse erfüllt zwei Aufgaben. Zum einen dichtet es den Eintritt des drahtförmigen Führungsteiles in den Kanülenansatz ab, so daß während des im wesentlichen reibungsfreien Vorschubes des Führungsteiles keine Luft in den punktierten Körperhohlraum eingeschleppt wird und zum anderen kann das Loch in dem Pfropfen radial so weit zusammengedrückt werden, daß der drahtförmige Führungsteil in der Stahlkanüle blockiert ist, so daß er weder vor- noch zurückrutschen kann. Letzteres bedeutet, daß nach Fixierung des Führungsteiles in der Stahlkanüle derart, daß seine Spitze im Auge des Stahlkanülenschliffs liegt, eine absolut zuverlässige Mandrinfunktion des Führungsteiles gegeben ist, d. h. daß kein Gewebe während der Punktion in das Lumen der Stahlkanüle eindringen kann. Da der Anwender den Führungsteil nicht festhalten muß, kann er sich dem Punktionsvorgang voll widmen, wodurch das Auffinden des Zielgebietes erleichtert und sicherer wird. Nach Lockerung des Stopfens wird der drahtförmige Führungsteil während des Vorschubes von dem gummielastischen Pfropfen abdichtend umschlossen, wobei der Verengungsgrad seines zentralen Durchlasses so gewählt ist, daß die Leichtgängigkeit des Führungsteilvorschubes praktisch nicht behindert wird. Sobald sich während des Führungsteilvorschubes durch Hindernisse in der Körperpassage Unterbrechungen ergeben, kann der Durchlaß durch die Quetschverschraubung wieder sehr stark verengt werden, und der Pfropfen hält den Führungsteil in der jeweiligen Position solange fest, bis das Hindernis z. B. durch Drehen oder Verkanten des Gesamtinstrumentes überwunden ist und der Führungsteil weiter vorgeschoben werden kann. Hierdurch werden unnötige Belastungen des Patienten infolge wiederholter Punktion vermieden.

Bei Benutzung als Punktionsbesteck muß das Gerät in der Lage sein, den Punktionserfolg anzuzeigen. Zu diesem Zweck ist in vorteilhafter Ausgestaltung der Erfindung in der Wand des Gehäuses unterhalb des Pfropfens eine Öffnung ausgebildet, an die ein Schlauch mit einem endseitigen Anschlußstück angeschlossen ist. Nach erfolgter Punktion eines Körperhohlraumes mit in der Stahlkanüle fixiertem Führungsteil strömt Körperfluid durch den Ringraum zwischen Führungsteil und Stahlkanüleninnenwand zurück, wird von dem wasserdicht abgesperrten Dichtungselement an einem axialen Austritt gehindert und verläßt das Gehäuse durch den Schlauch. Das Erscheinen von Körperfluid in dem Schlauch zeigt das Punktionsergebnis an. Durch angepaßte Bemessung des Ringraumes in bezug auf den Fluiddruck in dem zu punktierenden und zu katheterisierenden Körperhohlraum läßt sich die Anzeige des Punktionserfolges durch heraustropfendes Körperfluid beschleunigen. Außerdem ist es möglich, an das Anschlußstück des Schlauches eine Spritze anzusetzen und Körperfluid abzuziehen, um vor Einführung eines Katheters z. B. das Nierenbecken zu entwässern. Ferner kann man mit einer an das Anschlußstück des Schlauches angesetzten Spritze während der Punktion durch den Ringraum in der Stahlkanüle an dem Führungsteil vorbei beispielsweise NaCl-Lösung, die mit Röntgenkontrastmitteln oder mit gerinnungshemmenden Substanzen versetzt sein kann, in den Körperhohlraum injizieren.

Das Gehäuse und der Schlauch bestehen vorteilhafterweise aus transparentem Kunststoff. Der Führungsteil kann als Seldingerdraht, Lunderquist-Draht, Seil-Litze, Polymerdraht, als mit Polymermaterial überzogener drahtförmiger Teil, als vorne geschlossener Schlauch oder starres Metallrohr bzw. Stab ausgebildet sein.

In der Zeichnung ist ein Ausführungsbeispiel der Erfindung schematisch dargestellt. Es zeigen :

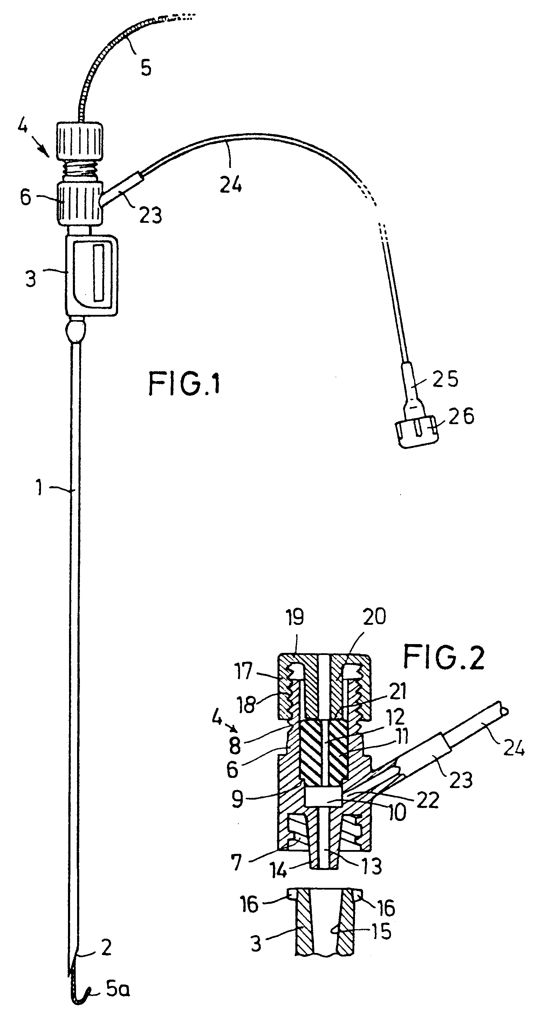

Fig. 1 eine Draufsicht auf ein Punktions- und Einführungsbesteck und

Fig. 2 die Abdichtungs- und Fixierungsvorrichtung im Längsschnitt im vergrößerten Maßstab.

Das Punktions- und Einführungsbesteck für einen Katheter, Dilatator, den Innenteil eines Dilatatorsets, eine Knopfsonde oder dergleichen besteht aus einer Stahlkanüle 1 mit angeschliffener Spitze 2 und einem Kanülenansatz 3, aus der Abdichtungs- und Fixierungsvorrichtung 4 und aus einem Führungsteil 5, der bei dem gewählten Beispiel ein Seldingerdraht mit gekrümmter Spitze 5a ist. Der Führungsteil 5 ist beträchtlich länger als die Stahlkanüle 1 und sein Außendurchmesser ist etwas kleiner als das Lumen der Stahlkanüle 1, so daß ein Ringraum zum Durchlaß von Fluid entsteht.

Die Abdichtungs- und Fixierungsvorrichtung 4 weist ein hülsenförmiges Gehäuse 6 mit zylindrischem Hohlraum von kreisförmigem Querschnitt auf, an dessen einem Ende ein Innengewinde 7 ausgebildet ist, während sich an seinem anderen Ende ein Außengewinde 8 befindet. Zwischen den beiden Enden des Gehäuses 6 ist über dem Innengewinde 7 in dem Hohlraum eine Ringschulter 9 ausgebildet, so daß der Hohlraum einen längeren Abschnitt größeren Durchmessers und einen kürzeren Abschnitt kleineren Durchmessers erhält. Auf der nach außen gerichteten Fläche der Ringschulter 9 liegt ein zylindrischer dicker, gummielastischer Pfropfen 11, der in dem größeren Hohlraumabschnitt eingepaßt ist und mit einem kurzen Fortsatz in den kleineren Hohlraumabschnitt ragt. Der Pfropfen 4 ist von einem zentralen Durchlaß 12 durchsetzt, welcher mit einem Kanal 13 in einem Außenkonus 14 im Bereich des Innengewindes 7 fluchtet. Der Außenkonus 14 greift in einen Innenkonus 15 des Kanülenansatzes 3 ein, wenn radiale Nocken 16 am Rand des Kanülenansatzes 3 in das Innengewinde 7 des Gehäuses 6 eingeschraubt sind und das Gehäuse 6 auf dem Kanülenansatz 3 festsitzt.

Auf das Außengewinde 8 des Gehäuses 6 ist ein Schraubring 17 mit Innengewinde 18 aufschraubbar. Der Schraubring 17 ist durch eine ringförmige Bodenplatte 19 abgeschlossen, an der ein nach innen gerichteter koaxialer Hohlstutzen 20 befestigt ist. Der Hohlstutzen 20 endet innerhalb des Schraubringes 17 und sein gerader Rand 21 wirkt mit der Außenfläche des gummielastischen Pfropfens 11 des Dichtungselementes zusammen, wenn der Schraubring 17 mehr oder weniger auf das Gehäuse 6 aufgeschraubt wird. Wenn der drahtförmige Führungsteil 5 durch den Schraubring 17, den Hohlstutzen 20, den zentralen Durchlaß 12 und den Kanal 13 hindurch in die Stahlkanüle 1 eingeführt ist, kann durch axiales Zusammenquetschen des gummielastischen Pfropfens 11 mittels des axial beweglichen Hohlstutzens 20 der zentrale Durchlaß 12 des gummielastischen Pfropfens 11 radial mehr oder weniger verengt werden. Ersteres hat zur Folge, daß der Führungsteil 5 vollständig blockiert werden kann, so daß er nicht mehr verschiebbar ist und das Gehäuse 6 wasserdicht verschlossen ist. Geringere Durchlaßverengung bewirkt, daß der Führungsteil 5 abgedichtet verschiebbar ist, so daß Lufteintritt in das Gehäuse 6 beim Vorschieben des Führungsteiles 5 vermieden wird.

In der Wand des Gehäuses 6 ist im Bereich eines freien Gehäusehohlraumes 10 zwischen den inneren Enden des Pfropfens 11 und des Außenkonus 14 eine Öffnung 22 ausgebildet, an die ein kleiner Seitenstutzen 23 angesetzt ist, an dem ein Ende eines dünnen Schlauches 24 befestigt ist. Das freie Ende des Schlauches 24 trägt ein Anschlußstück 25 zur Verbindung mit einer nicht gezeichneten Spritze. Das Anschlußstück 25 ist zunächst mittels einer Schraubkappe 26 verschlossen.

Das dargestellte Punktions- und Einführungsbesteck wird wie folgt angewendet :

Zunächst wird der Schraubring 17 so eingestellt, daß der Hohlstutzen 20 den Pfropfen 11 praktisch nicht verformt, so daß der zentrale Durchlaß 12 den Pfropfen 11 im wesentlichen seinen ursprünglichen Durchmesser aufweist und der Führungsteil 5 ungehindert frei verschiebbar ist. In diesem Zustand der Abdichtungs- und Fixierungsvorrichtung 4 wird der Führungsteil 5 in der Stahlkanüle 1 so justiert, daß die Spitze 5a mit dem hinteren Rand des Kanülenschliffs 2 abschließt und der Eintritt von Gewebe in das Lumen der Stahlkanüle 1 während der Punktion verhindert wird. Sodann wird der Schraubring 17 angezogen, wobei der Rand des Hohlstutzens 20 axial gegen den gummielastische Pfropfen 11 drückt und ihn so verformt, daß der Durchlaß 12 radial verengt wird, bis der Führungsteil 5 fest eingespannt ist und sich nicht mehr bewegen läßt. Mit Hilfe der nicht gezeichneten Spritze an dem Anschlußstück 25 wird der Schlauch 24 mit einer NaCl-Lösung, die z. B. Röntgenkontrastmittel enthalten kann, gefüllt und es wird der Körperhohlraum punktiert. Dabei wird beobachtet, ob Flüssigkeit aus dem Schlauch 24 abläuft oder sich abziehen bzw. injizieren läßt. Der jeweilige Status zeigt das Funktionsergebnis an. Anschließend wird der Schraubring 17 soweit gelockert, daß der Pfropfen 11 den Führungsteil 5 nicht mehr festhält, sondern ihn nur noch abdichtend umschließt, während er vorgeschoben wird. Wenn die Spitze 5a des Führungsteiles 5 die vorgesehene Position innerhalb des Körperhohlraumes eingenommen hat, wird die Stahlkanüle 1 mit dem Gehäuse 6 über den Führungsteil 5 zurückgezogen und es wird ein Katheter, ein Dilatator, ein Innenteil eines Dilatatorsets, eine Knopfsonde oder dergleichen über den Führungsteil 5 in den Körperhohlraum eingeführt.

## Patentansprüche

1. Punktions- und Einführungsbesteck für einen Katheter oder dergleichen, bestehend aus einer an ihrem patientenfernen Ende einen Kanülenansatz (3) aufweisenden Stahlkanüle (1), in der sich ein drahtförmiger Führungsteil (5) gröberer Länge befindet, dessen Außendurchmesser zur Bildung eines Ringraumes kleiner ist als das Lumen der Stahlkanüle (1) und der eine gekrumpte Spitze (5a) aufweist,

aus einer Abdichtungsvorrichtung (4), die an dem Kanülenansatz (3) angeordnet ist und einen in einem Gehäuse (6) untergebrachten, als Dichtungselement dienenden, gelochten gummielastischen Pfropfen (11) mit einem zentralen Durchlaß (12) für den Führungsteil (5) aufweist, der auf einer Ringschulter (9) des Gehäuses (6) liegt und mittels eines auf das Gehäuse (6) aufgeschraubten Druckkörpers (17,20) zusammenquetschbar ist, welcher einen gegen den gummielastischen Pfropfen (11) axial andrückbaren Hohlstutzen (20) aufweist, der den zentralen Durchlaß (12) des Pfropfens (11) radial zusammenquetscht, um den drahtförmigen Führungsteil (5) zu blockieren,

und aus einer in der Wand des Gehäuses (6) unterhalb des Pfropfens (11) ausgebildeten Öffnung (22), an die ein Schlauch (24) mit einem endseitigen Anschlußstück (25) angeschlossen ist.

2. Punktions- und Einführungsbesteck nach Anspruch 1, dadurch gekennzeichnet, daß der Hohlstutzen (20) an einem Schraubring (17) ausgebildet ist.

3. Punktions- und Einführungsbesteck nach Anspruch 1, dadurch gekennzeichnet, daß das Gehäuse (6) und der Schlauch (24) aus transparentem Kunststoff bestehen.

4. Punktions- und Einführungsbesteck nach einem der Ansprüche 1 bis 3, dadurch gekennzeichnet, daß das Gehäuse (6) mit dem Kanülenansatz (3) lösbar verbunden ist.

5. Punktions- und Einführungsbesteck nach einem der Ansprüche 1 bis 4, dadurch gekennzeichnet, daß der Führungsteil (5) als Seldingerdraht, Lunderquistdraht, Seil-Litze, Polymerdraht, als mit Polymermaterial überzogener drahtförmiger Teil, als vorne geschlossener Schlauch oder als starres Metallrohr bzw. Stab ausgebildet ist.

## Claims

1. Puncture and insertion set for a catheter or the like comprising a steel cannula (1) having at its distal end a cannula hub (3), said steel cannula containing a wire-type guide member (5) of a greater length, the outer diameter of which is smaller than the lumen of said steel cannula (1) so that an annular space is formed, said guide member having a curved tip (5a),

a sealing means (4) provided at said cannula hub (3) and including a perforated rubber-elastic plug (11) disposed in a housing (6) and serving as a sealing element, said plug resting on an annular shoulder (9) of the housing (6) and having a central passage (12) for said guide member (5), said plug being adapted to be squeezed by means of a pressure body (17,20) screwed on said housing (6), which pressure body comprises a hollow socket (20) adapted to be urged axially against said rubber-elastic plug (11) and radially squeezing said central passage (12) of said plug (11) in order to block said wire-type guide member (5), and

an aperture (22) formed in the wall of said housing (6) beneath said plug (11), for connecting thereto a hose (24) with an end-sided connecting member (25).

2. Puncture and insertion set according to claim 1, characterized in that said hollow socket (20) is formed at a screw ring (17).

3. Puncture and insertion set according to claim 1, characterized in that said housing (6) and said hose (24) are made of transparent plastics.

4. Puncture and insertion set according to one of claims 1 to 3, characterized in that said housing (6) is detachably connected to said cannula hub (3).

5. Puncture and insertion set according to one of claims 1 to 4, characterized in that said guide member (5) is a Seldinger wire, Lunderquist wire, cable cord, polymeric wire, a wire-type element coated with polymeric material, a hose closed at its front end, or a rigid metal tube or rod.

## Revendications

1. Ensemble de ponction et d'introduction pour un cathéter et analogue, constitué par une canule en acier, qui comporte sur son extrémité éloignée du patient un embout de canule (3) et dans laquelle se trouve disposée une partie de guidage en forme de fil (5) d'une longueur plus grande, dont le diamètre extérieur en vue de former un espace annulaire est inférieur à celui de l'ouverture de la canule en acier (1) et qui comporte une pointe recourbée (5a),

par un dispositif d'étanchéité (4), qui est monté sur l'embout de canule (3) et comporte un bouchon (11) perforé présentant l'élasticité du caoutchouc, logé dans un boîtier (6) et servant d'organe d'étanchéité, qui est disposé sur un épaulement annulaire (9) du boîtier (6) et dans lequel est ménagé un passage central (12) pour la partie de guidage (5), et peut être comprimé par serrage à l'aide d'un organe de compression (17, 20) vissé sur le boîtier (6) et possédant une tubulure creuse (20), qui peut être repoussée axialement contre le bouchon (11) présentant l'élasticité du caoutchouc et resserre radialement le passage central (12) du bouchon (11) afin de bloquer la partie de guidage en forme de fil (5),

et par une ouverture (22) ménagée dans la paroi du boîtier (6) au-dessous du bouchon (11), et à laquelle est raccordé un tuyau (24) possédant un élément de raccordement (25) situé à son extrémité.

2. Ensemble de ponction et d'introduction selon la revendication 1, caractérisé en ce que la tubulure creuse (20) est formée sur une bague vissable (17).

3. Ensemble de ponction et d'introduction selon la revendication 1, caractérisé en ce que le boîtier (6) et le tuyau (24) sont réalisés en une matière plastique transparente.

4. Ensemble de ponction et d'introduction selon l'une des revendications 1 à 3, caractérisé en ce que le boîtier (6) est relié de façon amovible à l'embout de canule (3).

5. Ensemble de ponction et d'introduction selon l'une des revendications 1 à 4, caractérisé en ce que la partie de guidage (5) est réalisée sous la forme d'un fil Seldinger, d'un fil Lunderquist, d'un fil toronné, d'un fil en polymère, d'un élément en forme de fil recouvert d'un matériau polymère, d'un tuyau fermé à sa partie avant ou bien d'un tube ou d'une barre métallique rigide.

FIG.1

FIG.2